# EUROPÄISCHE PATENTANMELDUNG

(11) **EP 4 342 403 A1**
(43) Veröffentlichungstag der Anmeldung: **27.03.2024**
(21) Anmeldenummer: 22197150.0
(22) Anmeldetag: 22.09.2022
(51) Int. Cl.: A61B 18/12, A61B 18/14, A61B 90/30

(54) **VERFAHREN ZUR ERMITTLUNG EINES ZUSTANDS EINES LICHTWELLENLEITERS EINES ELEKTROCHIRURGISCHEN INSTRUMENTS UND SYSTEM MIT EINEM ELEKTROCHIRURGISCHEN INSTRUMENT**

(71) Anmelder: Erbe Elektromedizin GmbH, 72072 Tübingen (DE)
(72) Erfinder: REITERER, Markus, 2620 Loipersbach (AT); DRAGOSTINOFF, Nikolaus, 1230 Wien (AT); UNGER, Alexanderr, 2431 Enzersdorf/Fischa (AT)
(74) Vertreter: Rüger Abel Patentanwälte PartGmbB

(57) **Zusammenfassung**

Die Erfindung betrifft ein Verfahren (V) und ein System (10), das dazu eingerichtet ist, einen Zustand eines Lichtwellenleiters (23) eines elektrochirurgischen Instruments (12) zu ermitteln im Hinblick auf seine Übertragungscharakteristik (U) für Licht. Dazu kann das System (10) eine Auswerteeinheit (27) mit einer Lichtquelle (34) und einer Lichtanalyseeinheit (28) aufweisen, die optisch mit dem Lichtwellenleiter (23) gekoppelt werden kann. Das von der Lichtquelle emittierte Emissionslicht (Le) wird in den Lichtwellenleiter (23) eingekoppelt, an dessen distalem Ende reflektiert und zurück zur Lichtanalyseeinheit (28) übertragen und dort als Empfangslicht (Lr) empfangen. Das Empfangslicht (Lr) wird in Beziehung gesetzt zum Emissionslicht (Le) und daraus kann eine Übertragungscharakteristik (U) ermittelt werden. Wird dies vor der erstmaligen Benutzung des elektrochirurgischen Instruments (12) und anschließend wenigstens ein weiteres Mal ausgeführt, können dadurch Veränderungen in der Übertragungscharakteristik (U) erkannt und daraus wiederum auf den Zustand des Lichtwellenleiters (23) geschlossen werden.

## Beschreibung

Die Erfindung betrifft ein Verfahren zur Ermittlung eines Zustands eines Lichtwellenleiters eines elektrochirurgischen Instruments. Die Erfindung betrifft außerdem ein System aufweisend ein elektrochirurgisches Instrument.

Mittels elektrochirurgischen Instrumenten kann menschliches oder tierisches Gewebe durch Einwirkung mittels einer elektrischen Entladung bearbeitet werden. Hierzu weist das elektrochirurgische Instrument wenigstens eine Elektrode auf. Beispielsweise kann mittels des elektrochirurgischen Instruments koaguliert oder geschnitten werden.

EP 2 815 695 A1 beschreibt ein elektrochirurgisches Instrument mit einer Elektrode. Bei der Bearbeitung von Gewebe entsteht Licht an der Elektrode, das mittels eines Lichtwellenleiters zur spektralen Analyse an eine Auswerteeinheit weitergeleitet wird. In der Auswerteeinheit kann anhand des Lichts der Gewebetyp des behandelten Gewebes ermittelt werden.

Ein Verfahren zur Bestimmung des Gewebetyps ist ferner aus EP 3 964 822 A1 bekannt. Dort wird das über den Lichtwellenleiter vom elektrochirurgischen Instrument zu einer Auswerteeinheit übertragene Licht bewertet und ein Transmissionsgrad ermittelt. Dadurch ist es möglich, die Verschmutzung des elektrochirurgischen Instruments am Lichteintrittsfenster zu ermitteln und dadurch die Zuverlässigkeit bei der Bestimmung des Gewebetyps zu ermitteln.

In DE 198 40 346 A1 wird ein Verfahren und eine Einrichtung zur Diagnose und Überwachung der Übertragungsgüte eines faseroptischen Systems beschrieben. Ein Laserbehandlungsinstrument ist über einen Lichtwellenleiter mit einer Laserlichtquelle verbunden, der das Laserinstrument mit Laserlicht versorgt. Der Lichtwellenleiter wird kontinuierlich überwacht, um Schäden zu erkennen. Mittels einer zusätzlichen Testlichtquelle kann Testlicht in den Lichtwellenleiter eingekoppelt und reflektiertes Licht empfangen und ausgewertet werden, um die Übertragungsgüte zu bestimmen. Das empfangene Licht wird dabei über einen Polarisationsstrahlteiler geleitet, um Anteile an reflektiertem Licht zu entfernen, die vor dem Einkoppeln des Testlichts in den Lichtwellenleiter entstehen. Ein ähnliches Verfahren ist auch aus EP 3 949 887 A1 bekannt. Dort weist das Laserbehandlungsinstrument zur Reflexion von eingekoppeltem Licht eine Reflexionseinheit am distalen Ende des Lichtwellenleiters auf.

Bei dem in EP 2 823 278 A2 beschriebenen Verfahren zur Bewertung der Integrität eines Lichtwellenleiters wird eine Reflexion an einem Faserende von der ermittelten Gesamtreflexion bei der Einkopplung von Licht in den Lichtwellenleiter getrennt, um eine kalibrierte Intensitätsmessung für die Integritätsbewertung des Lichtwellenleiters zu erhalten.

US 5 965 877 A schlägt vor, die Integrität eines Lichtwellenleiters durch Verwendung von Licht mit unterschiedlichen Wellenlängen zu bewerten. Während des Einsatzes verwendeten Arbeitslichts kann Testlicht in Lichtwellenleiter eingekoppelt werden, das am entgegengesetzten Faserende lumineszierendes Material zur Lumineszenz anregt, wobei dieses Licht eine weitere unterschiedliche Wellenlänge aufweist. Das durch die Lumineszenz erzeugte Reflexionslicht wird durch den Lichtwellenleiter zurückübertragen und kann ausgewertet werden, um auf die Integrität des Lichtwellenleiters zu schließen.

CN 209264547 U beschreibt einen Faserkoppler mit drei Anschlüssen. Ein Anschluss ist mit einer Lichtquelle, ein weiterer Anschluss mit einem Detektor und ein dritter Anschluss mit einer Gasmesszelle verbunden. Licht kann von Lichtquelle zur Gasmesszelle übertragen, dort reflektiert und zurück zum Detektor übertragen werden. Für den Lichtweg zu der Gasmesszelle und für den Lichtweg von der Gasmesszelle zurück zum Detektor werden unterschiedliche Fasern des Lichtwellenleiters verwendet.

Weitere Verfahren und Einrichtungen zur Bewertung des Zustandes eines Lichtwellenleiters sind in US 4 716 288 A oder US 5 219 345 A beschrieben.

Ferner beschreiben US 7 132 645 B2 und EP 0 926 479 A1 Systeme und Verfahren, um die Verluste in einem einen Lichtwellenleiter aufweisenden Lichtweg zu ermitteln.

Ausgehend vom Stand der Technik kann es als Aufgabe der vorliegenden Erfindung angesehen werden, ein Verfahren zur Ermittlung eines Zustands eines Lichtwellenleiters eines elektrochirurgischen Instruments zu schaffen, das sich mit einfachen Mitteln kostengünstig realisieren lässt. Außerdem ist es eine Aufgabe der Erfindung, ein System bereitzustellen, mittels dem das Verfahren einfach und kostengünstig durchgeführt werden kann.

Diese Aufgaben werden durch ein Verfahren mit den Merkmalen des Patentanspruches 1 sowie ein System mit den Merkmalen des Patentanspruches 10 gelöst.

Das erfindungsgemäße Verfahren ist dazu eingerichtet, den Zustand eines Lichtwellenleiters eines elektrochirurgischen Instruments zu ermitteln. Das elektrochirurgische Instrument weist wenigstens eine Elektrode auf. Es kann als monopolares oder bipolares elektrochirurgisches Instrument ausgebildet sein.

Zur Versorgung mit elektrischer Energie der wenigstens einen Elektrode ist das elektrochirurgische Instrument mit einer Energiequelle eines Versorgungsgeräts elektrisch verbunden. Hierzu kann das elektrochirurgische Instrument wenigstens einen elektrischen Leiter aufweisen, der jeweils die Elektrode mit der Energiequelle verbindet.

Das elektrochirurgische Instrument weist außerdem einen Lichtwellenleiter auf, der ein distales Ende sowie ein proximales Ende aufweist. Das distale Ende ist beispielsweise an einem Griffstück des elektrochirurgischen Instruments befestigt und kann eine distale Stirnfläche aufweisen, die als Lichteintrittsfenster zur Erfassung von Licht an der wenigstens einen Elektrode dient. Über den Lichtwellenleiter kann das elektrochirurgische Instrument optisch mit einer Auswerteeinheit verbunden werden. Die Auswerteeinheit kann Bestandteil des Versorgungsgeräts sein.

Zur Beurteilung des Zustands des Lichtwellenleiters, insbesondere der Verschmutzung des Lichteintrittsfensters bzw. des distalen Endes des Lichtwellenleiters wird das elektrochirurgische Instrument zunächst in einem Ausgangszustand bereitgestellt. Im Ausgangszustand kann das elektrochirurgische Instrument neu sein oder bei Mehrweginstrumenten gereinigt bzw. sterilisiert sein. Das elektrochirurgische Instrument kann sowohl als Einweginstrument als auch als wiederverwendbares Mehrweginstrument ausgeführt sein.

Das elektrochirurgische Instrument wird im Ausgangszustand mit der Energiequelle elektrisch verbunden und mit der Auswerteeinheit optisch verbunden. Anschließend wird eine Übertragungscharakteristik ermittelt, die bei einem elektrochirurgischen Instrument, das sich im nicht beschädigten und nicht verschmutzten Ausgangszustand befindet, als Referenzcharakteristik dient.

Anschließend kann das elektrochirurgische Instrument zur Behandlung von tierischem oder menschlichem Gewebe eines Patienten eingesetzt werden. Hierzu wird das elektrochirurgische Instrument aktiviert, wobei die wenigstens eine Elektrode mit elektrischer Energie der Energiequelle versorgt wird. An der wenigstens einen Elektrode entsteht eine elektrische Entladung, beispielsweise ein Funken oder ein Lichtbogen, mittels dem auf das Gewebe eingewirkt werden kann, beispielsweise um das Gewebe zu koagulieren oder zu schneiden.

Nach dem Deaktivieren des elektrochirurgischen Instruments - wodurch die Versorgung der wenigstens einen Elektrode mit elektrischer Energie abgeschaltet wird - wird erneut eine Übertragungscharakteristik als aktuelle Zustandscharakteristik ermittelt. Dieses Ermitteln einer aktuellen Zustandscharakteristik kann immer durchgeführt werden, wenn ein vorgegebenes Kriterium erfüllt ist. Das vorgegebene Kriterium kann beispielsweise in einem der nachfolgenden Fälle erfüllt sein:
- jeweils nach einmaligem Aktivieren und Deaktivieren des elektrochirurgischen Instruments;
- jeweils nach einer vorgegebenen Anzahl an Aktivierungen und Deaktivierungen des elektrochirurgischen Instruments, wobei die Anzahl mindestens 2 ist;
- nach einer vorgegebenen Gesamtaktivierungszeitdauer des elektrochirurgischen Instruments durchgeführt werden.

Anschließend kann jeweils durch Vergleich der aktuelle Zustandscharakteristik mit der ursprünglich ermittelten Referenzcharakteristik der Zustand und insbesondere der Verschmutzungszustand des distalen Endes des Lichtwellenleiters ermittelt werden.

Für diese Ermittlung des aktuellen Zustands des Lichtwellenleiters sind keine aufwendigen Einrichtungen erforderlich. Das zum Behandeln vorgesehene elektrochirurgische Instrument wird im Ausgangszustand und zusätzlich nach Gebrauch im Hinblick auf seine Übertragungscharakteristik geprüft, wodurch sich die Änderung des Zustands gegenüber dem Ausgangszustand erkennen lässt. Wenn beispielsweise durch Verschleiß, Verschmutzung, Beschädigung oder dergleichen eine Abweichung des Zustands des Lichtwellenleiters vom Ausgangszustand erkannt wird, können Maßnahmen eingeleitet werden. Beispielsweise kann eine Reinigung oder ein Austausch des elektrochirurgischen Instruments veranlasst werden. Hierzu kann mittels der Auswerteeinheit das Ausgeben einer entsprechenden Meldung an eine Bedienperson veranlasst werden.

Für das Ermitteln einer Übertragungscharakteristik als Referenzcharakteristik oder als aktuelle Zustandscharakteristik wird wie folgt vorgegangen:

Eine Lichtquelle der Auswerteeinheit emittiert Licht, das als Emissionslicht bezeichnet werden kann. Das Emissionslicht wird zumindest teilweise in das proximale Ende des Lichtwellenleiters eingekoppelt und dort in Richtung des distalen Endes weitergeleitet. Zumindest ein Teil des am distalen Ende ankommenden Emissionslichts wird dort reflektiert und zurück in Richtung des proximalen Endes durch den Lichtwellenleiter geleitet. Am proximalen Ende wird zumindest ein Teil des reflektierten Lichts ausgekoppelt und an die Auswerteeinheit übermittelt. Das in der Auswerteeinheit ankommende Licht kann als Empfangslicht bezeichnet werden. Durch eine Auswertung basierend auf dem Emissionslicht und dem Empfangslicht kann die Übertragungscharakteristik ermittelt werden. Beispielsweise kann ein Reflexionsgrad ermittelt werden, indem die Intensität des Empfangslichts mit der Intensität des Emissionslichts verglichen wird. Die Übertragungscharakteristik kann wellenlängenabhängig sein. Sie kann zum Beispiel ein Reflexionsspektrum bzw. einen Reflexionsgrad für mehrere Wellenlängen beschreiben.

Das Verfahren ist vorzugsweise dazu eingerichtet, Licht, das durch Funken oder Lichtbogenbildung an der wenigstens einen Elektrode entsteht - beispielsweise während eines elektrochirurgischen Eingriffs an menschlichem oder tierischem Gewebe - zumindest teilweise am distalen Ende des Lichtwellenleiters zu empfangen und an die Auswerteeinheit zu übertragen. In der Auswerteeinheit kann das empfangene Licht analysiert werden, insbesondere durch eine spektrale Analyse mittels einer Lichtanalyseeinheit der Auswerteeinheit, beispielsweise eines Spektrometers. Anhand der Auswertung des empfangenen Lichts kann wenigstens ein Gewebeparameter ermittelt werden, beispielsweise ein Gewebetyp. Insbesondere ist die spektrale Zusammensetzung des Lichts bei der Behandlung von unterschiedlichen Gewebetypen verschieden, wobei Gewebetypen, wie Fettgewebe, Muskelgewebe, Knochengewebe, etc. voneinander unterschieden werden können. Es ist dabei möglich, der Bedienperson bzw. dem Operateur den jeweils aktuell behandelten Gewebetyp anzuzeigen.

Es ist vorteilhaft, wenn die Lichtquelle nur dann zum Emittieren von Emissionslicht aktiviert wird, wenn an der wenigstens einen Elektrode keine elektrische Energie anliegt. Dadurch kann sichergestellt werden, dass bei der Erfassung von Licht durch eine elektrische Entladung an der Elektrode und die Übertragung des Lichts vom distalen Ende des Lichtwellenleiters zur Auswerteeinheit keine Beeinflussung durch das Emissionslicht der Lichtquelle erfolgt. Beispielsweise ist dann eine Gewebeanalyse während der Behandlung des Gewebes verbessert.

Es ist vorteilhaft, wenn das Emissionslicht der Lichtquelle Lichtwellenlängen im Bereich von weniger als 380 nm aufweist. Das Emissionslicht der Lichtquelle kann mehrere Maxima bei unterschiedlichen Lichtwellenlängen aufweisen, wobei die Maxima unterschiedliche Intensitäten haben können. Beispielsweise kann ein Hauptmaxima bei Lichtwellenlängen von größer als 400 nm auftreten, etwa im Bereich zwischen 450 nm und 460 nm. Das Emissionslicht kann optional ein Nebenmaximum im Bereich von 340 nm bis 360 nm aufweisen.

Als Lichtquelle kann beispielsweise eine kostengünstige Leuchtdiode verwendet werden, beispielsweise eine Leuchtdiode des Typs MLEROY-A1-0000-000501.

Vorzugsweise hat die Auswerteeinheit zur Analyse von Licht eine Lichtanalyseeinheit, die insbesondere als Spektrometer und vorzugsweise als Spaltspektrometer kostengünstig ausgeführt werden kann. Zur optischen Kopplung der Auswerteeinheit mit einem Lichtwellenleiter des Versorgungsgerätes oder dem Lichtwellenleiter des elektrochirurgischen Instruments kann eine Kopplungseinheit vorhanden sein. Die Kopplungseinheit kann als 2:1-Koppler ausgebildet sein und somit drei Anschlüsse aufweisen: einen ersten Anschluss für die Lichtquelle, einen zweiten Anschluss für das die Lichtanalyseeinheit und einen dritten Anschluss für den Lichtwellenleiter des Versorgungsgerätes oder des elektrochirurgischen Instruments.

Bei einem ersten Ausführungsbeispiel ist ein Lichtwellenleiter der Versorgungsgerätes an einem Ende an den dritten Anschluss der Kopplungseinheit angeschlossen und am entgegengesetzten, anderen Ende mit einem Geräteanschluss des Versorgungsgeräts verbunden. An den Geräteanschluss (beispielsweise Buchse) kann der Lichtwellenleiter des Instruments, mithilfe eines Verbindungselements (beispielsweise Stecker) angeschlossen werden.

In Abwandlung dazu kann bei einem zweiten Ausführungsbeispiel die Kopplungseinheit Bestandteil des Geräteanschlusses des Versorgungsgeräts sein, so dass der Lichtwellenleiter des Instruments mittels des Verbindungselements an den dritten Anschluss der Kopplungseinheit angeschlossen werden kann.

Bei allen Ausführungsbeispielen kann eine Verschmutzung am distalen Ende des Lichtwellenleiters des Instruments oder eine andere Beeinträchtigung des Lichtwellenleiters des Instruments festgestellt werden. Zusätzlich kann beim ersten Ausführungsbeispiel auch eine Verschmutzung oder eine andere Beeinträchtigung am Geräteanschluss festgestellt werden.

Bei der Verwendung einer solchen Kopplungseinheit können der erste Anschluss und der zweite Anschluss optisch voneinander getrennt sein, so dass eine direkte optische Übertragung vom ersten Anschluss an den zweiten Anschluss allein mittels der Kopplungseinheit ausgeschlossen ist. Eine Übertragung von Licht vom ersten Anschluss an den zweiten Anschluss oder umgekehrt ist insbesondere nur dann möglich, wenn einer der Lichtwellenleiter optisch mit dem dritten Anschluss verbunden ist.

Bei der Verwendung einer solchen Kopplungseinheit kann Emissionslicht am ersten Anschluss der Kopplungseinheit eingekoppelt werden. In der Auswerteeinheit kann geprüft werden, ob Empfangslicht empfangen wird und/oder ein Merkmal des Empfangslichts (z.B. Intensität oder Lichtstärke) ausgewertet werden. Wird Empfangslicht empfangen, kann zumindest beim zweiten Ausführungsbeispiel in der Auswerteeinheit festgestellt werden, dass ein elektrochirurgisches Instrument mit seinem Lichtwellenleiter an den dritten Anschluss der Kopplungseinheit (als Bestandteil des Geräteanschlusses) angeschlossen ist. Beim ersten Ausführungsbeispiel kann durch die Auswertung des Merkmals des Empfangslichts geprüft werden, ob das elektrochirurgische Instrument an den Geräteanschluss angeschlossen ist. Beispielsweise kann die Intensität oder Lichtstärke des Empfangslichts mit der Intensität oder Lichtstärke des Emissionslichts verglichen werden. Die Intensität oder Lichtstärke des Empfangslichts nimmt zu, wenn zusätzlich an dem Übergang vom Lichtwellenleiter des Versorgungsgerätes zum Lichtwellenleiter des Instruments und/oder am distalen Ende des Lichtwellenleiters des Instruments jeweils ein Teil des Emissionslichts reflektiert wird.

Durch wiederholtes Emittieren von Emissionslicht und Einkoppeln des Emissionslichts am ersten Anschluss kann somit geprüft und festgestellt werden, ob das elektrochirurgische Instrument an die Kopplungseinheit angeschlossen ist, so dass anschließend die Referenzcharakteristik ermittelt werden kann.

Alternativ hierzu kann das Ermitteln der Referenzcharakteristik auch von der Bedienperson über eine Bedienschnittstelle ausgelöst werden, beispielsweise dann wenn eine automatische Erkennung eines an das Versorgungsgerät angeschlossenen Instruments nicht implementiert ist.

Ein erfindungsgemäßes System kann vorzugsweise zur Durchführung irgendeines Ausführungsbeispiels des vorstehend beschriebenen Verfahrens eingerichtet sein. Das System weist ein Versorgungsgerät mit einer Energiequelle zur Versorgung der wenigstens einen Elektrode des elektrochirurgischen Instruments auf. Außerdem hat das System eine Auswerteeinheit. Die Auswerteeinheit kann Bestandteil des Versorgungsgeräts sein oder auch separat vom Versorgungsgerät ausgeführt sein. Die Auswerteeinheit hat wiederum eine Lichtquelle und eine Lichtanalyseeinheit (z.B. Spektrometer) zur Analyse von Licht. Wie erläutert, kann als Spektrometer vorzugsweise ein kostengünstiges Spaltspektrometer verwendet. Mittels des Spalts des Spaltspektrometers kann das Empfangslicht in seine spektralen Anteile aufgeteilt werden.

Das elektrochirurgische Instrument hat einen Lichtwellenleiter, der mittels einer Kopplungseinheit optisch mit der Auswerteeinheit und mithin sowohl mit der Lichtquelle, als auch mit der Lichtanalyseeinheit gekoppelt ist. Dabei ist die Lichtquelle optisch an einen ersten Anschluss der Kopplungseinheit, die Lichtanalyseeinheit optisch an einen zweiten Anschluss der Kopplungseinheit und der Lichtwellenleiter des Instruments mittelbar (z.B. über einen Lichtwellenleiter des Versorgungsgeräts) oder unmittelbar optisch an einen dritten Anschluss der Kopplungseinheit angeschlossen.

Es sei an dieser Stelle darauf hingewiesen, dass die Nummerierung der Anschlüsse lediglich zur Unterscheidung dient und keine Einschränkung für die Anzahl der Anschlüsse oder für eine Reihenfolge oder Priorisierung der Anschlüsse darstellt.

Die Kopplungseinheit ist derart ausgebildet, dass keine unmittelbare optische Übertragung zwischen dem ersten Anschluss und dem zweiten Anschluss erfolgt. Der erste Anschluss und der zweite Anschluss sind optisch voneinander getrennt und jeweils separat optisch mit dem dritten Anschluss der Kopplungseinheit verbunden.

Die Ausgestaltung dieser Kopplungseinheit und der daran angeschlossenen Bestandteile, insbesondere die diesbezüglich nachfolgend beschriebenen Merkmale, ist ein separater Aspekt der Erfindung, der auch unabhängig von der erfindungsgemäßen Ausgestaltung des Verfahrens und des Systems realisiert werden kann.

Es ist bevorzugt, wenn die Kopplungseinheit ein Faserbündel mit wenigstens einer ersten optischen Faser und wenigstens einer zweiten optischen Faser aufweist. Die wenigstens eine erste optische Faser verbindet den ersten Anschluss der Kopplungseinheit optisch mit dem dritten Anschluss der Kopplungseinheit. Die wenigstens eine zweite optische Faser verbindet den zweiten Anschluss der Kopplungseinheit optisch mit dem dritten Anschluss der Kopplungseinheit. Vorzugsweise kann genau eine erste optische Faser vorhanden sein. Weiter vorzugsweise sind mehrere zweite optische Fasern vorhanden. Am dritten Anschluss und in einem sich an den dritten Anschluss anschließenden Bereich der Kopplungseinheit ist die erste optische Faser vorzugsweise zentral angeordnet und die mehreren zweiten optischen Fasern können in Umfangsrichtung um die erste optische Faser verteilt und insbesondere gleichmäßig verteilt angeordnet sein. Es ist vorteilhaft, wenn zumindest drei zweite optische Fasern vorhanden sind. Bei einer bevorzugten Ausführungsform sind vier zweite optische Fasern vorhanden.

Es ist vorteilhaft, wenn die optische Achse der ersten optischen Faser entlang der optischen Achse des Lichtwellenleiters ausgerichtet ist, wenn der Lichtwellenleiter an den dritten Anschluss der Kopplungseinheit angeschlossen ist.

Es ist vorteilhaft, wenn die Kernquerschnittsflächen der wenigstens einen ersten optischen Faser und der wenigstens einen zweiten optischen Faser jeweils kleiner sind als die Kernquerschnittsfläche des an den dritten Anschluss angeschlossenen Lichtwellenleiters. Die Kernquerschnittsfläche des an den dritten Anschluss angeschlossenen Lichtwellenleiters hat eine Umfangskontur, innerhalb der sämtliche Kernquerschnittsflächen der wenigstens einen ersten optischen Faser und der wenigstens einen zweiten optischen Faser angeordnet sind.

Vorteilhafterweise hat die wenigstens eine erste optische Faser eine Kernquerschnittsfläche, die kleiner ist als die Kernquerschnittsfläche der wenigstens einen zweiten optischen Faser. Beispielsweise kann die Kernquerschnittsfläche der wenigstens einen ersten optischen Faser maximal 25% der Kernquerschnittsfläche der wenigstens einen zweiten optischen Faser betragen. Zusätzlich oder alternativ kann die Kernquerschnittsfläche der wenigstens einen zweiten optischen Faser maximal 25% der Kernquerschnittsfläche des Lichtwellenleiters betragen.

Die Auswerteeinheit kann eine Anordnung zur Modenmischung aufweisen. Die Anordnung zur Modenmischung ist beim ersten Ausführungsbeispiel zwischen dem Geräteanschluss beim zweiten Ausführungsbeispiel zwischen der Kopplungseinheit und der Auswerteeinheit angeordnet. Das zwischen der Auswerteeinheit und der Kopplungseinheit übertragene Licht wird daher in Bezug auf die vorhandenen Moden gemischt. Dies betrifft sowohl das zur Kopplungseinheit übertragene Emissionslicht, als auch das von der Kopplungseinheit zur Auswerteeinheit übertragene Empfangslicht.

Vorteilhafte Ausgestaltungen der Erfindung ergeben sich aus den abhängigen Ansprüchen, der Beschreibung und der Zeichnung. Nachfolgend werden bevorzugte Ausführungsbeispiele der Erfindung anhand der beigefügten Zeichnung im Einzelnen erläutert. In der Zeichnung zeigen:

Figur 1 ein Blockschaltbild eines ersten Ausführungsbeispiels eines erfindungsgemäßen Systems aufweisend eine Energiequelle, eine Auswerteeinrichtung und ein elektrochirurgisches Instrument,

Figur 2 eine blockschaltbildähnliche Prinzipdarstellung des elektrochirurgischen Instruments, das gemäß des ersten Ausführungsbeispiels nach Figur 1 über eine Kopplungseinheit an die Auswerteeinheit angeschlossen ist,

Figur 3 ein Blockschaltbild eines zweiten Ausführungsbeispiels eines erfindungsgemäßen Systems aufweisend eine Energiequelle, eine Auswerteeinrichtung und ein elektrochirurgisches Instrument,

Figur 4 eine blockschaltbildähnliche Prinzipdarstellung des elektrochirurgischen Instruments, das gemäß des zweiten Ausführungsbeispiels nach Figur 3 über eine Kopplungseinheit an die Auswerteeinheit angeschlossen ist,

Figur 5 eine Prinzipdarstellung der Anordnung von wenigstens einer ersten Faser und wenigstens einer zweiter Faser der Kopplungseinheit aus Figur 2 an einem dritten Anschluss der Kopplungseinheit,

Figur 6 eine Prinzipdarstellung einer proximalen Stirnfläche eines Lichtwellenleiters des elektrochirurgischen Instruments,

Figur 7 eine Prinzipdarstellung der Anordnung von mehreren zweiten optischen Fasern der Kopplungseinheit gemäß der Figuren 2 und 3 an einem zweiten Anschluss der Kopplungseinheit aus Figur 2,

Figur 8 eine allgemeine Prinzipdarstellung einer bevorzugten Anordnungsmöglichkeit von optischen Fasern an einer beliebigen Kopplungsstelle,

Figur 9 ein beispielhaftes Spektrum von Emissionslicht einer Lichtquelle der Auswerteeinheit,

Figur 10 beispielhafte Übertragungscharakteristiken eines Lichtwellenleiters bzw. eines elektrochirurgischen Instruments in Abhängigkeit von einer Lichtwellenlänge und

Figur 11 ein Flussdiagramm eines Ausführungsbeispiels eines erfindungsgemäßen Verfahrens.

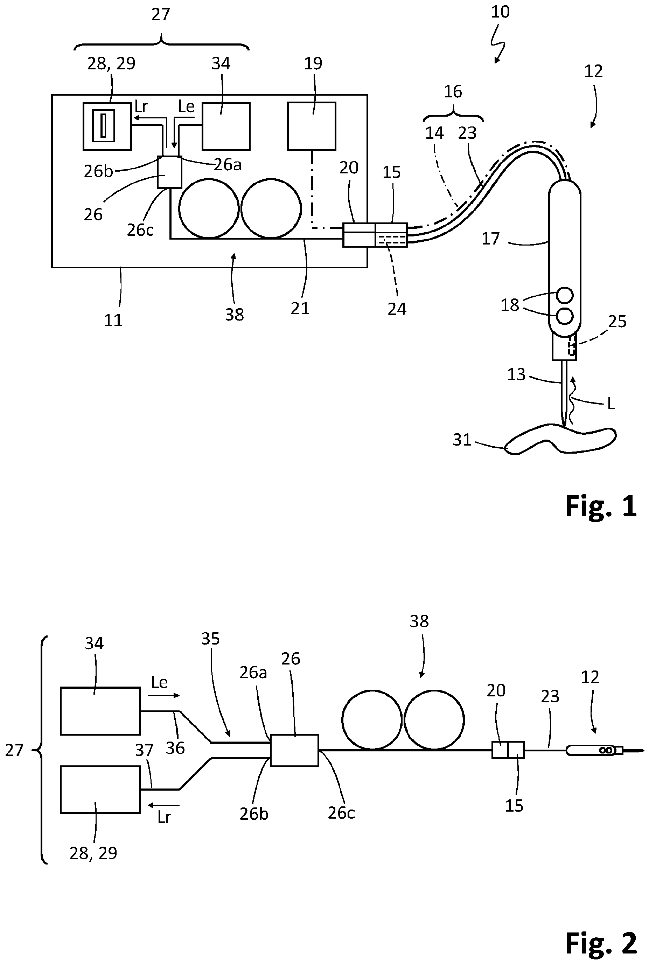

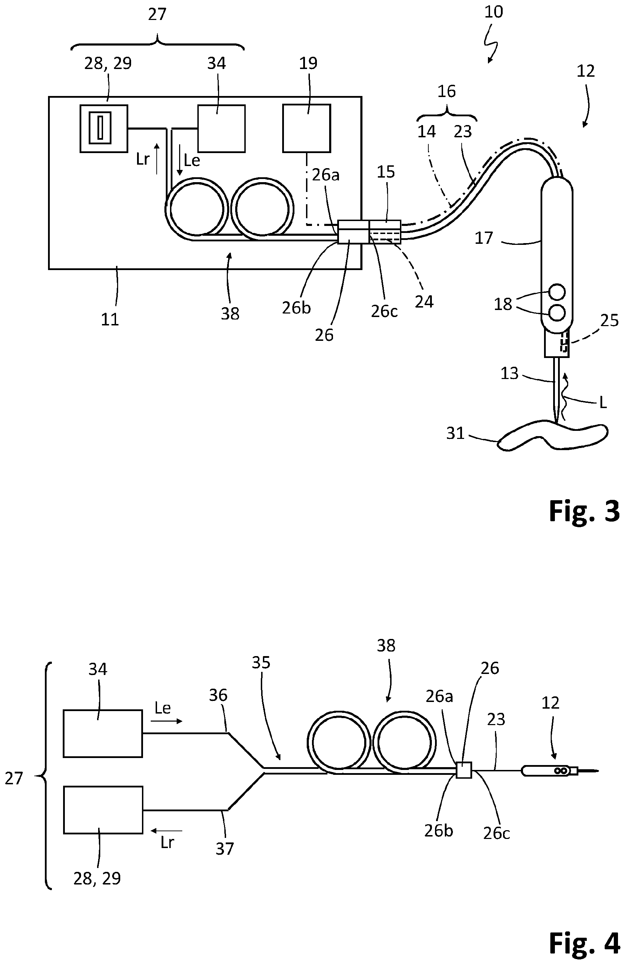

In den Figuren 1 und 2 ist schematisch ein erstes Ausführungsbeispiel eines Systems 10 und in den Figuren 3 und 4 ist ein zweites Ausführungsbeispiel eines Systems 10 veranschaulicht. Das System 10 weist ein Versorgungsgerät 11 und ein daran angeschlossenes bzw. anschließbares elektrochirurgisches Instrument 12 auf.

Das elektrochirurgische Instrument 12 kann als monopolares oder als bipolares Instrument ausgeführt sein und weist wenigstens eine Elektrode 13 auf. Die wenigstens eine Elektrode 13 ist über einen zugeordneten elektrischen Leiter 14 mit einem Verbindungselement 15 elektrisch verbunden. Der elektrische Leiter 14 kann Bestandteil eines Kabels 16 sein. Das Kabel 16 erstreckt sich zwischen dem Verbindungselement 15 und einem Griffstück 17 des elektrochirurgischen Instruments 12.

Die wenigstens eine Elektrode 13 ist am Griffstück 17 befestigt. Am Griffstück 17 sind beim Ausführungsbeispiel ein oder mehrere Bedienelemente 18 vorhanden, mittels denen das elektrochirurgische Instrument 12 aktivierbar ist und deaktivierbar ist. Zusätzlich zu dem wenigstens einen Bedienelement 18 am Griffstück 17 könnte auch ein separater Fußschalter oder dergleichen vorhanden sein, um das elektrochirurgische Instrument 12 zu aktivieren oder zu deaktivieren.

Über das Versorgungsgerät 11 wird die wenigstens eine Elektrode 13 im aktivierten Zustand mit elektrischer Energie versorgt. Hierfür hat das Versorgungsgerät 11 eine Energiequelle 19. Die Energiequelle 19 ist elektrisch mit einem Geräteanschluss 20 verbunden. Der Geräteanschluss 20 ist dazu eingerichtet, mit dem Verbindungselement 15 verbunden zu werden. Dadurch kann über den wenigstens einen elektrischen Leiter 14 eine elektrische Verbindung zwischen der Energiequelle 19 und der wenigstens einen Elektrode 13 hergestellt werden.

Das elektrochirurgische Instrument 12 weist außerdem einen Lichtwellenleiter 23 auf. Der Lichtwellenleiter 23 des elektrochirurgischen Instruments 12 hat ein proximales Ende 24, das mit dem Verbindungselement 15 verbunden ist. Ein distales Ende 25 des Lichtwellenleiters 23 ist am Griffstück 17 befestigt, vorzugsweise mittels einer stoffschlüssigen Verbindung, beispielsweise einer Klebeverbindung. Das distale Ende 25 könnte zusätzlich oder alternativ auch kraftschlüssig und/oder formschlüssig am Griffstück 17 befestigt sein. Eine Stirnfläche des Lichtwellenleiters 23 am distalen Ende 25 dient als Lichtaufnahmefenster und ist der Elektrode 13 zugewandt. Durch das distale Ende 25 eintretendes Licht kann über den Lichtwellenleiter 23 zum proximalen Ende 24 geleitet werden.

Das proximale Ende 24 des Lichtwellenleiters 23 des elektrochirurgischen Instruments 12 kann über das Verbindungselement 15 und den Geräteanschluss 20 optisch mit einer Kopplungseinheit 26 und über die Kopplungseinheit 26 optisch mit einer Auswerteeinheit 27 gekoppelt werden. Die Kopplungseinheit 26 kann im Inneren des Gehäuses des Versorgungsgerätes 11 angeordnet und über einen Lichtwellenleiter 21 des Versorgungsgerätes 11 mit dem Geräteanschluss 20 optisch verbunden sein (erstes Ausführungsbeispiel nach Figuren 1 und 2) oder Bestandteil des Geräteanschlusses 20 sein (zweites Ausführungsbeispiel nach Figuren 3 und 4). Durch Verbinden des Verbindungselements 15 mit dem Geräteanschluss 20 kann in beiden Fällen eine optische Kopplung zwischen dem proximalen Ende 24 und der Kopplungseinheit 26 und dadurch auch mit der Auswerteeinheit 27 hergestellt werden.

Alternativ zum veranschaulichten Ausführungsbeispiel ist es auch möglich, die elektrische Verbindung und die optische Verbindung zwischen dem elektrochirurgischen Instrument 12 und dem Versorgungsgerät 11 durch mehrere separate Verbindungselemente 15 und separate Geräteanschlüsse 20 auszuführen.

Die Auswerteeinheit 27 ist beim Ausführungsbeispiel Bestandteil des Versorgungsgeräts 11. Bei einer abgewandelten Ausführungsform könnte die Auswerteeinheit 27 auch separat vom Versorgungsgerät 11 in einem eigenständigen Gehäuse angeordnet sein.

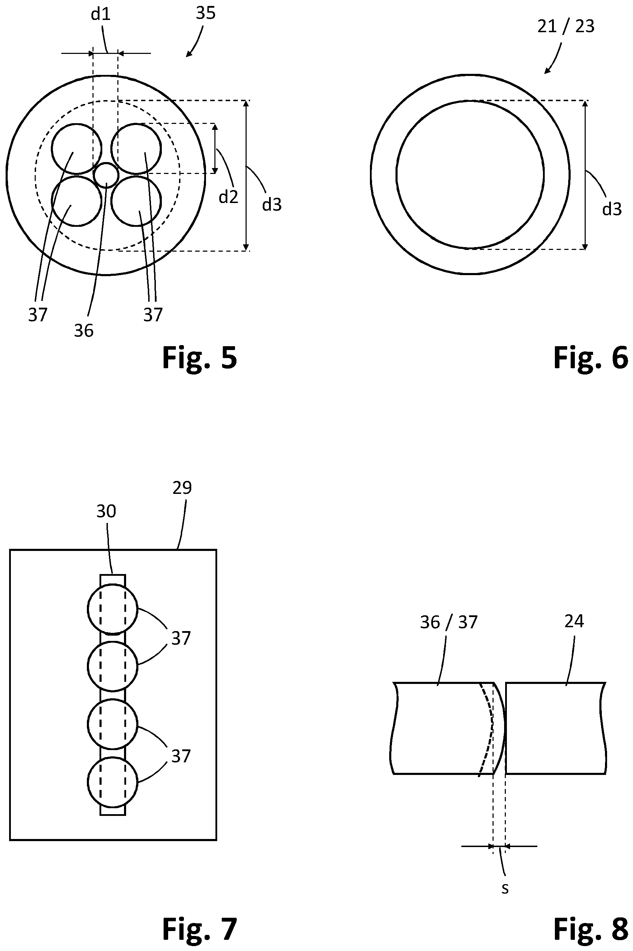

Die Auswerteeinheit 27 weist zur Auswertung von Licht, das vom elektrochirurgischen Instrument 12 übertragen wurde, eine Lichtanalyseeinheit 28 auf, die beim Ausführungsbeispiel als Spektrometer 29 und insbesondere als Spaltspektrometer ausgeführt ist. Mittels eines Spalts 30 des Spektrometers 29 (Figur 7) kann das empfangene Licht in seine enthaltenen Lichtwellenlängen λ unterteilt werden und die Intensitäten der enthaltenen Lichtwellenlängen λ können einzeln ausgewertet werden.

Während der Verwendung des elektrochirurgischen Instruments 12 bei der Behandlung von menschlichem oder tierischem Gewebe 31 entsteht an der wenigstens einen Elektrode 13 eine elektrische Entladung, beispielsweise ein Funken und/oder ein Lichtbogen. Das dabei entstehende Licht L kann teilweise in die Stirnfläche am distalen Ende 25 des Lichtwellenleiters 23 eintreten und wird über das proximale Ende 24 am Verbindungselement 15 über die Kopplungseinheit 26 an die Lichtanalyseeinheit 28 der Auswerteeinheit 27 weitergeleitet. Dort kann durch die Analyse des Lichts, insbesondere die spektrale Analyse des Lichts, wenigstens ein Gewebeparameter des behandelten Gewebes 31 ermittelt werden, beispielsweise der Gewebetyp. Dadurch können unterschiedliche Gewebetypen, wie etwa Muskelgewebe, Fettgewebe, Knochengewebe, usw. voneinander unterschieden werden und der Bedienperson bzw. dem Operateur über eine geeignete Schnittstelle angezeigt werden.

Beim Ausführungsbeispiel gehört zur Auswerteeinheit 27 außerdem eine Lichtquelle 34. Die Lichtquelle 34 und die Lichtanalyseeinheit 28 sind mittels eines Faserbündels 35 mit der Kopplungseinheit 26 optisch verbunden. Das Faserbündel 35 weist wenigstens eine erste optische Faser 36 und wenigstens eine zweite optische Faser 37 auf. Die wenigstens eine erste optische Faser 36 ist an einem Ende optisch mit der Lichtquelle 34 verbunden und an einem anderen Ende optisch mit einem ersten Anschluss 26a der Kopplungseinheit 26 verbunden. Die wenigstens eine zweite optische Faser 37 ist an einem Ende optisch mit der Lichtanalyseeinheit 28 und an einem anderen Ende optisch mit einem zweiten Anschluss 26b der Kopplungseinheit 26 verbunden. Der Lichtwellenleiter 21 des Versorgungsgeräts 11 (Figuren 1 und 2) oder der Lichtwellenleiter 23 des Instruments 12 (Figuren 3 und 4) ist optisch mit einem dritten Anschluss 26c der Kopplungseinheit 26 verbunden.

Beispielsgemäß weist das Faserbündel 35 genau eine erste optische Faser 36 und mehrere zweite optische Fasern 37 auf, vorzugsweise mindestens drei zweite optische Fasern 37 und beim Ausführungsbeispiel vier zweite optische Fasern 37. Innerhalb des Faserbündels 35 erstreckt sich die erste optische Faser 36 entlang einer Längsmittelachse des Faserbündels 35, wie es schematisch in Figur 5 veranschaulicht ist. Die mehreren zweiten optischen Fasern 37 sind in Umfangsrichtung verteilt um die erste optische Faser 36 angeordnet. Zur optischen Verbindung mit der Auswerteeinheit 27 bzw. der Kopplungseinheit 26 wird das Faserbündel 35 im Bereich seines jeweiligen Endes aufgetrennt.

Wie es aus Figur 5 hervorgeht, ist die Kernquerschnittsfläche und beispielsgemäß der Kerndurchmesser der wenigstens einen optischen Faser 36 kleiner als der Kernquerschnitt bzw. der Kerndurchmesser der wenigstens einen zweiten optischen Faser 37. Die Kernquerschnittsfläche bzw. die Kerndurchmesser der wenigstens einen ersten optischen Faser 36 und der wenigstens einen zweiten optischen Faser 37 sind wiederum kleiner als die Kernquerschnittsfläche bzw. der Kerndurchmesser des an den dritten Anschluss 26c angeschlossenen Lichtwellenleiters 21, 23 (Figur 6).

Zur besseren Unterscheidung sind beim Ausführungsbeispiel der Kerndurchmesser der wenigstens einen ersten optischen Faser 36 als erster Durchmesser d1, der Kerndurchmesser der wenigstens einen zweiten optischen Faser als zweiter Durchmesser d2 und der Kerndurchmesser des Lichtwellenleiters 21 bzw. 23 als dritter Durchmesser d3 bezeichnet.

Beispielsgemäß ist der dritte Durchmesser d3 mindestens so groß oder größer als die Summe aus dem ersten Durchmesser d1 und zweimal dem zweiten Durchmesser d2. Dadurch ist es möglich, die eine erste optische Faser 36 und die vier zweiten optischen Fasern 37 derart anzuordnen, dass sie sich vollständig innerhalb einer Kreisfläche befinden, die durch den dritten Durchmesser d3 definiert ist (Figur 5).

Beispielsweise kann der dritte Durchmesser d3 600 µm, der zweite Durchmesser d2 200 µm und der erste Durchmesser d1 100 µm betragen.

Die Lichtwellenleiter 21, 23 ist beispielsgemäß als Multimodenfaser (MMF) ausgeführt. Zusätzlich oder alternativ können auch die wenigstens eine optische Faser 36 und/oder die wenigstens eine zweite optische Faser 37 jeweils als Multimodenfaser ausgebildet sein. In diesem Fall ist es vorteilhaft, wenn zwischen der Auswerteeinheit 27 und der Kopplungseinheit 26 eine Anordnung 38 zur Modenmischung vorhanden ist (Figuren 1 bis 4). Die Anordnung 38 zur Modenmischung kann beispielsweise durch das Anordnen des Lichtwellenleiters 21 des Versorgungsgerätes 11 (Figuren 1 und 2) oder des Faserbündels 35 (Figuren 3 und 4) in wenigstens einem Bogen und/oder wenigstens einer Schleife erreicht werden. Vorzugsweise werden mehrere Bögen oder Schleifen in räumlich unterschiedlichen Ebenen angeordnet, beispielsweise in drei rechtwinkelig zueinander ausgerichteten Ebenen. Die Schleifen können kreisrund, elliptisch, in Form einer "8" oder dergleichen angeordnet sein.

Wie es schematisch in Figur 7 veranschaulicht ist, werden die zweiten optischen Fasern 37 entlang einer Linie nebeneinander in Erstreckungsrichtung des Spalts 30 angeordnet. Auf diese Weise lässt sich ein Großteil der Spaltfläche durch die mehreren zweiten optischen Fasern überdecken und eine Großlichtmenge durch den Spalt 30 in das Spektrometer 29 einkoppeln. Dadurch wird die spektrale Analyse verbessert und ist noch bei geringen Lichtmengen möglich, die am distalen Ende 25 des Lichtwellenleiters 23 beim Betrieb des elektrochirurgischen Instruments 12 empfangen werden.

In Figur 8 ist schematisch eine bevorzugte Art von optischer Kopplung zwischen zwei Faserenden veranschaulicht. Diese optische Kopplung kann an jeder Stelle des Systems 10 verwendet werden, an der zwei Faserenden optisch miteinander verbunden werden sollen. Die Faserenden berühren sich vorzugsweise oder liegen sich mit einem Abstand s gegenüber (gestrichelt dargestellt). Der optionale Abstand s stellt den minimalen Abstand zwischen den Stirnflächen der Faserenden dar. Die optischen Achsen der beiden Faserenden sind vorzugsweise parallel oder deckungsgleich. Die Stirnfläche des einen Faserendes ist eine ebene Fläche, während die Stirnfläche des anderen Faserendes eine konvexe Fläche ist. Die konvexe Stirnfläche kann eine polierte Fläche sein. Die ebene Stirnfläche des jeweils anderen Faserendes kann eine polierte ebene Fläche sein oder eine nicht nachbehandelte Bruchfläche. Das Faserende mit der ebenen Stirnfläche kann beispielsweise einer der Lichtwellenleiter 21, 23 sein, während das andere Faserende mit der konvexen Stirnfläche das Faserende der ersten Faser 36 und/oder einer zweiten Faser 37 sein kann. Es ist auch möglich, umgekehrt die Faserenden in der wenigstens einen ersten optischen Faser 36 und/oder der wenigstens einen zweiten optischen Faser 37 eben auszugestalten und die Stirnfläche am proximalen Ende 24 eines der Lichtwellenleiter 21, 23 konvex auszuführen.

Die Lichtquelle 34 emittiert Licht, das als Emissionslicht Le bezeichnet werden kann. Als Lichtquelle 34 kann beispielsweise eine Leuchtdiode, beispielsweise eine UV-Leuchtdiode verwendet werden, die zumindest auch im UV-Wellenlängenbereich Emissionslicht Le emittiert.

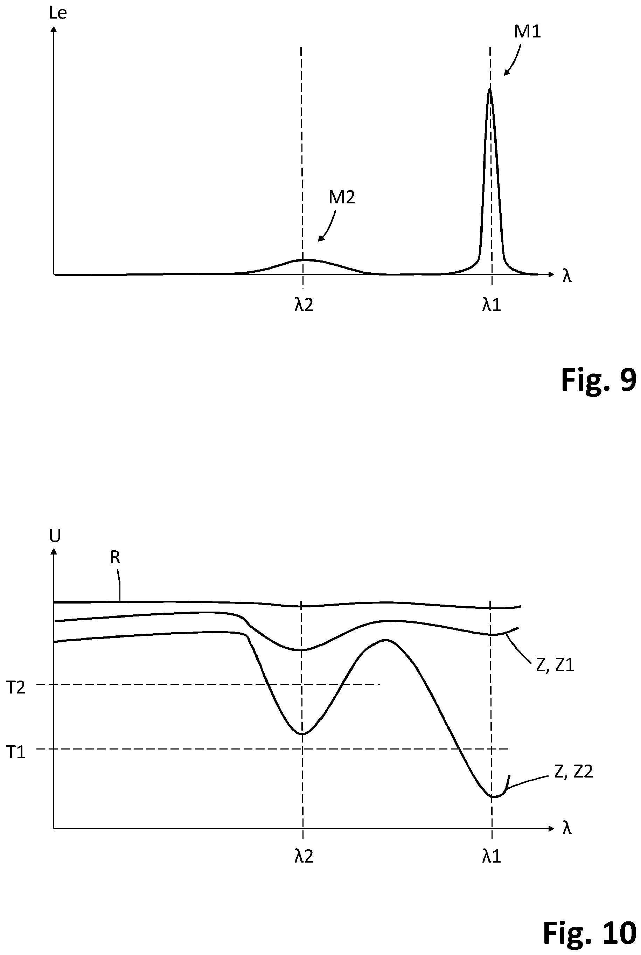

Ein beispielhaftes Spektrum des Emissionslichts Le ist in Figur 9 veranschaulicht. Das Emissionslicht Le enthält vorzugsweise Lichtwellenlängen λ in einem Bereich von kleiner als 380 nm. Beim Ausführungsbeispiel hat das Spektrum des Emissionslichts Le ein erstes Maximum M1 bei einer ersten Lichtwellenlänge λ1 und ein zweites Maximum M2 bei einer zweiten Lichtwellenlänge λ2. Die erste Lichtwellenlänge λ1 liegt im Bereich von 400 nm bis 500 nm und beim Ausführungsbeispiel im Bereich von 450 nm bis 460 nm. Die zweite Lichtwellenlänge λ2 ist kleiner als 380 nm und liegt beispielsgemäß im Bereich zwischen 340 nm und 360 nm, beispielsweise bei etwa 350 nm. Ein derartiges Spektrum lässt sich mit einer kostengünstigen Leuchtdiode erzielen. Das erste Maximum M1 ist beispielsgemäß das Hauptmaximum und das zweite Maximum M2 das Nebenmaximum des Spektrums des Emissionslichts Le.

Zusätzlich oder alternativ zum zweiten Maximum M2 könnte das Emissionslicht Le auch Licht mit einer Wellenlänge im Bereich zwischen 200 nm und 260 nm enthalten, was weitere Vorteile bei der Auswertung der Übertragungscharakteristik U des elektrochirurgischen Instruments 12 bzw. des Lichtwellenleiters 23 mit sich bringen kann, da hier die Unterschiede in der Transmission von Licht abhängig von einem Verschmutzungsgrad des distalen Endes 25 besonders deutlich werden.

Das in Figur 9 dargestellte uns beispielsgemäß verwendete Spektrum des Emissionslichts Le ist lediglich beispielhaft. Das Spektrum des Emissionslichts Le könnte auch eine andere spektrale Zusammensetzung aufweisen.

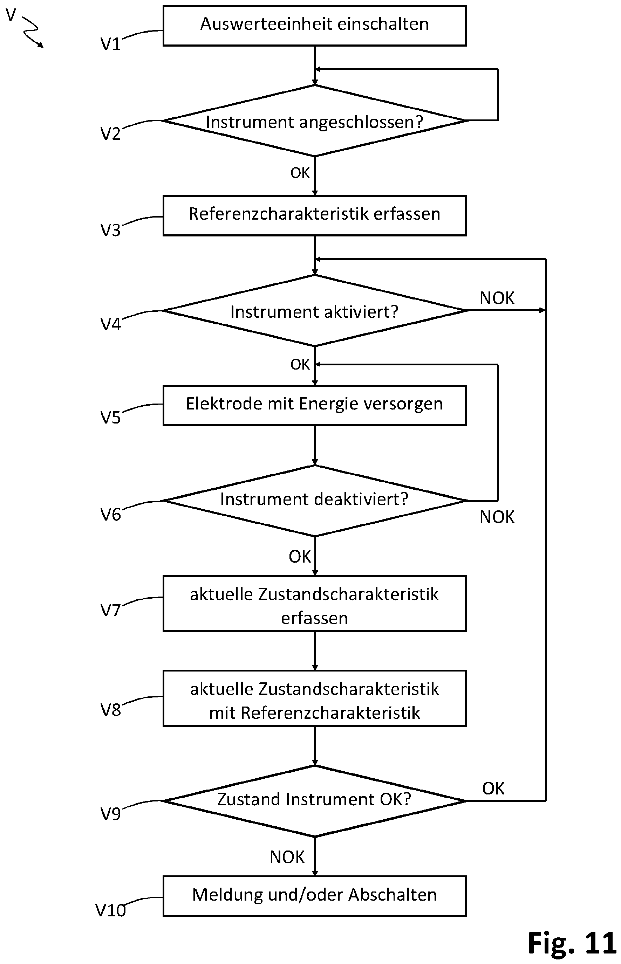

In Figur 11 ist ein Ausführungsbeispiel eines Verfahrens V veranschaulicht. Das Verfahren V ist dazu eingerichtet, einen Zustand des Lichtwellenleiters 23 anhand dessen Übertragungscharakteristik U zu ermitteln, insbesondere eine Verschmutzung des distalen Endes 25 und/oder andere Schäden oder Beeinträchtigungen zu erkennen, die die Lichtübertragung beeinflussen. Mit zunehmender Verschmutzung oder bei Auftreten von anderen Schäden oder Verschleiß am Lichtwellenleiter 23 kann das Spektrum des übertragenen Lichts beeinflusst werden, so dass beispielsweise mittels der Lichtanalyseeinheit 28 die Ermittlung eines Gewebeparameters nicht mehr mit ausreichender Sicherheit möglich ist. Mittels des Verfahrens V kann eine solche Beeinträchtigung erkannt und eine geeignete Maßnahme veranlasst werden, beispielsweise eine Reinigung des elektrochirurgischen Instruments 12 bzw. des distalen Endes 25 des Lichtwellenleiters 23 oder auch ein Austausch des elektrochirurgischen Instruments 12.

In einem ersten Verfahrensschritt V1 wird die Auswerteeinheit 27 eingeschaltet. In einem optionalen zweiten Verfahrensschritt V2 wird geprüft, ob ein neues oder gereinigtes oder sterilisiertes elektrochirurgisches Instrument 12 - also ein elektrochirurgisches Instrument 12 in einem Ausgangszustand - an die Auswerteeinheit 27 angeschlossen ist. Erst, wenn das elektrochirurgische Instrument 12 angeschlossen ist (Verzweigung OK aus dem zweiten Verfahrensschritt V2) wird das Verfahren in einem dritten Verfahrensschritt V3 fortgesetzt. Das erfolgte Anschließen des Instruments 12 an das Versorgungsgerät 11 kann alternativ von einer Bedienperson mittels einer geeigneten Bedienschnittstelle bestätigt werden, um das Verfahren im dritten Verfahrensschritt V3 fortzusetzen.

Im dritten Verfahrensschritt V3 wird erstmals eine Übertragungscharakteristik U für das sich durch den Lichtwellenleiter 23 geleitete Licht ermittelt. Die Übertragungscharakteristik U kann zusätzlich abhängig sein von der optischen Kopplung zwischen dem Verbindungselement 15 und der Kopplungseinheit 26 und/oder der optional vorhandenen Anordnung 38 zur Modenmischung und/oder wenigstens einem weiteren optischen Einflussparameter. Die Übertragungscharakteristik U wird jedoch zumindest auch vom Zustand des Lichtwellenleiters 23 und insbesondere der Verschmutzung des distalen Endes 25 des Lichtwellenleiters 23 beeinflusst. Beim ersten Ausführungsbeispiel kann zusätzlich auch eine Verschmutzung des Geräteanschlusses 20 geprüft und ermittelt werden, da dadurch die Lichtreflexion an einem dem Geräteanschluss 20 zugeordneten Ende des Lichtwellenleiters 21 des Versorgungsgerätes 11 beeinflusst wird. Eine Verschmutzung am Geräteanschluss 20 kann von einem korrekt angeschlossenen und optisch gekoppelten Lichtwellenleiter 21 des Instruments 12 in der Lichtanalyseeinheit 28 unterschieden werden. Andere Einflussparameter sind beim Betrieb des Systems 10 im Wesentlichen konstant, so dass eine Veränderung der Übertragungscharakteristik U zumindest im Wesentlichen auf den Zustand des Lichtwellenleiters 23 zurückgeführt werden kann.

Nach dem Anschließen eines elektrochirurgischen Instruments 12 im Ausgangszustand (neu oder sterilisiert oder gereinigt) stellt die im dritten Verfahrensschritt V3 ermittelte Übertragungscharakteristik U eine Referenzcharakteristik R dar, wie sie beispielhaft in Figur 10 veranschaulicht ist. Ist das elektrochirurgische Instrument 12 im Ausgangszustand, in dem der Lichtwellenleiter 23 weder verschmutzt, noch beschädigt ist, wird das Licht durch den Lichtwellenleiter 23 beispielsgemäß im Wesentlichen unabhängig von der Lichtwellenlänge λ übertragen.

Um die Übertragungscharakteristik U zu ermitteln, wird beispielsgemäß wie folgt vorgegangen:

Die Lichtquelle 34 wird aktiviert und Emissionslicht Le wird mittels der Kopplungseinheit 26 (und den optional vorhandenen Lichtwellenleiter 21 des Versorgungsgerätes sowie den Geräteanschluss 20) zumindest teilweise in das proximale Ende 24 des Lichtwellenleiters 23 eingekoppelt. Dort wird das Licht bis zum distalen Ende 25 weitergeleitet und dort teilweise reflektiert. Das reflektierte Licht wird zurück in Richtung zum proximalen Ende 24 geleitet und dort mittels der Kopplungseinheit 26 zur Lichtanalyseeinheit 28 (z.B. Spektrometer 29) übermittelt. Das in der Lichtanalyseeinheit 28 ankommende Licht kann als Empfangslicht Lr bezeichnet werden. Das Empfangslicht Lr und das Emissionslicht Le werden dann in Beziehung zueinander gesetzt, um die Übertragungscharakteristik U zu ermitteln. Beim Ausführungsbeispiel werden die Intensitäten bei mehreren Lichtwellenlängen λ zueinander ins Verhältnis gesetzt, so dass die Übertragungscharakteristik U einen Reflexionsgrad der Lichtübertragung darstellt.

Nach dem Erfassen der Referenzcharakteristik R wird das Verfahren V in einem vierten Verfahrensschritt V4 fortgesetzt, in dem geprüft wird, ob das elektrochirurgische Instrument 12 zur Behandlung von Gewebe 31 aktiviert wurde. Das Aktivieren kann durch die Bedienperson bzw. dem Operateur beispielsweise mittels dem wenigstens einen Bedienelement 18 am Griffstück 17 veranlasst werden. Im aktivierten Zustand des elektrochirurgischen Instruments 12 (Verzweigung OK aus dem vierten Verfahrensschritt V4) wird die wenigstens eine Elektrode 13 mit elektrischer Energie versorgt, insbesondere wird eine Hochfrequenzspannung angelegt (fünfter Verfahrensschritt V5). Beispielsweise kann dann das Gewebe 31 mittels einer an der wenigstens einen Elektrode 13 erzeugten elektrischen Entladung behandelt werden, beispielsweise mittels Koagulation oder Dissektion.

In einem sechsten Verfahrensschritt V6 wird geprüft, ob das Instrument wieder deaktiviert wurde. Solange dies nicht der Fall ist (Verzweigung NOK aus dem sechsten Verfahrensschritt V6), wird die wenigstens eine Elektrode 13 weiterhin mit elektrischer Energie versorgt (fünfter Verfahrensschritt V5). Erst nach dem Deaktivieren des elektrochirurgischen Instruments 12 (Verzweigung OK aus dem sechsten Verfahrensschritt V6) wird das Verfahren in einem siebten Verfahrensschritt V7 fortgesetzt und erneut eine Übertragungscharakteristik U als Zustandscharakteristik Z erfasst. Beispiele für eine erste Zustandscharakteristik Z1 und eine zweite Zustandscharakteristik Z2 sind in Figur 10 schematisch dargestellt.

Nach dem Erfassen der aktuellen Zustandscharakteristik Z im siebten Verfahrensschritt V7 wird in einem achten Verfahrensschritt V8 die aktuelle Zustandscharakteristik Z mit der im dritten Verfahrensschritt V3 erfassten Referenzcharakteristik R verglichen bzw. in Beziehung gesetzt. Sind die Unterschiede zwischen der aktuellen Zustandscharakteristik Z und der Referenzcharakteristik R ausreichend klein, wird in einem neunten Verfahrensschritt V9 festgestellt, dass der Zustand des elektrochirurgischen Instruments die Fortsetzung des Betriebs gestattet (Verzweigung OK aus dem neunten Verfahrensschritt V9 zum vierten Verfahrensschritt V4).

Wird hingegen eine Abweichung der aktuellen Zustandscharakteristik Z von der Referenzcharakteristik R festgestellt, die ein vorgegebenes Kriterium erfüllt, wird ein die Fortsetzung des Betriebs nicht gestattender Zustand (beispielsweise Verschmutzung) erkannt (Verzweigung NOK aus dem neunten Verfahrensschritt V9). Eine solche Verschmutzung kann bei allen Ausführungsbeispielen am distalen Ende 25 des Lichtwellenleiters 21 des Instruments 12 und beim ersten Ausführungsbeispiel zusätzlich auch am Geräteanschluss 20 festgestellt werden. Dann kann in einem zehnten Verfahrensschritt V10 eine geeignete Maßnahme angefordert oder automatisch eingeleitet werden, beispielsweise eine Meldung an die Bedienperson bzw. den Operateur und/oder das Abschalten der Energieversorgung des elektrochirurgischen Instruments 12. Beispielsweise kann dann ein Austausch des elektrochirurgischen Instruments 12 oder eine Reinigung durchgeführt werden, um den Betrieb wieder aufzunehmen.

Im Falle der in Figur 10 gezeigten beispielhaften ersten Zustandscharakteristik Z1 sind die Unterschiede zur Referenzcharakteristik R ausreichend klein, so dass in einem neunten Verfahrensschritt V9 festgestellt wird, dass der Zustand des elektrochirurgischen Instruments gemäß der ersten Zustandscharakteristik Z1 die Fortsetzung des Betriebs gestattet. Beispielhaft ist in Figur 10 veranschaulicht, dass die zweite Zustandscharakteristik Z2 Abweichungen gegenüber der Referenzcharakteristik R aufweist, die eine nicht tolerierbare Veränderung angibt, beispielsweise Verschmutzung des distalen Endes 25 des Lichtwellenleiters 23.

Durch eine Verschmutzung sinkt beim Ausführungsbeispiel der Reflexionsgrad bei der ersten Lichtwellenlänge λ1 und der zweiten Lichtwellenlänge λ2 deutlich, an denen das Emissionslicht Le das erste Maximum M1 bzw. das zweite Maximum M2 aufweist. Beispielsweise können ein oder mehrere Schwellenwerte der Übertragungscharakteristik U definiert werden, die eine maximal zulässige Abweichung von der Referenzcharakteristik R definieren. Für unterschiedliche Lichtwellenlängen λ können jeweils unterschiedliche Schwellenwerte T1, T2 definiert werden, wie es beispielhaft in Figur 10 zu erkennen ist. Beispielsweise kann der ersten Lichtwellenlänge λ1 ein erster Schwellenwert T1 und der zweiten Lichtwellenlänge λ2 ein zweiter Schwellenwert T2 zugeordnet werden. Zusätzlich oder alternativ ist es auch möglich, anstatt eines konstanten Schwellenwerts für jede Lichtwellenlänge einen lichtwellenlängenabhängigen Schwellenwertverlauf zu definieren.

Die Erfindung betrifft ein Verfahren V und ein System 10, das dazu eingerichtet ist, einen Zustand eines Lichtwellenleiters 23 eines elektrochirurgischen Instruments 12 zu ermitteln im Hinblick auf seine Übertragungscharakteristik U für Licht. Dazu kann das System 10 eine Auswerteeinheit 27 mit einer Lichtquelle 34 und einer Lichtanalyseeinheit 28 aufweisen, die optisch mit dem Lichtwellenleiter 23 gekoppelt werden kann, insbesondere über ein Faserbündel 38 und eine Kopplungseinheit 26 gemäß eines weiteren unabhängigen erfindungsgemäßen Aspekts. Das von der Lichtquelle emittierte Emissionslicht Le wird in den Lichtwellenleiter 23 eingekoppelt, an dessen distalem Ende reflektiert und zurück zur Lichtanalyseeinheit 28 übertragen und dort als Empfangslicht Lr empfangen. Das Empfangslicht Lr wird in Beziehung gesetzt zum Emissionslicht Le und daraus kann eine Übertragungscharakteristik U ermittelt werden. Wird dies vor der erstmaligen Benutzung des elektrochirurgischen Instruments 12 und anschließend wenigstens ein weiteres Mal ausgeführt, können dadurch Veränderungen in der Übertragungscharakteristik U erkannt und daraus wiederum auf den Zustand des Lichtwellenleiters 23 geschlossen werden.

Der Lichtwellenleiter 23 des Instruments 12 ist insbesondere unmittelbar oder mittelbar über einen weiteren Lichtwellenleiter 21 des Versorgungsgerätes 11 an die Kopplungseinheit 26 angeschlossen und mit einem Faserbündel 35 optisch gekoppelt, das wenigstens eine optische Faser 36 und wenigstens eine optische Faser 37 aufweist. Die wenigstens eine erste optische Faser 36 verbindet die Kopplungseinheit 26 mit der Lichtquelle 34 und die wenigstens eine zweite optische Faser 37 verbindet die Kopplungseinheit 26 mit der Lichtanalyseeinheit 28.

### Bezugszeichenliste:

- 10: System
- 11: Versorgungsgerät
- 12: elektrochirurgisches Instrument
- 13: Elektrode
- 14: elektrischer Leiter
- 15: Verbindungselement
- 16: Kabel
- 17: Griffstück
- 18: Bedienelement
- 19: Energiequelle
- 20: Geräteanschluss
- 21: Lichtwellenleiter des Versorgungsgerätes

- 23: Lichtwellenleiter des elektrochirurgischen Instruments
- 24: proximalen Ende des Lichtwellenleiters des elektrochirurgischen Instruments
- 25: distalen Ende des Lichtwellenleiters des elektrochirurgischen Instruments
- 26: Kopplungseinheit
- 26a: erster Anschluss der Kopplungseinheit
- 26b: zweiter Anschluss der Kopplungseinheit
- 26c: dritter Anschluss der Kopplungseinheit
- 27: Auswerteeinheit
- 28: Lichtanalyseeinheit
- 29: Spektrometer
- 30: Spalt

- 34: Lichtquelle
- 35: Faserbündels
- 36: erste optische Faser
- 37: zweite optische Faser
- 38: Anordnung zur Modenmischung

- λ: Lichtwellenlänge
- λ1: erste Lichtwellenlänge
- λ2: zweite Lichtwellenlänge

- d1: erster Kerndurchmesser
- d2: zweiter Kerndurchmesser
- d3: dritter Kerndurchmesser
- L: Licht vom Gewebe
- Le: Emissionslicht
- Lr: Empfangslicht
- M1: erstes Maximum
- M2: zweites Maximum
- R: Referenzcharakteristik
- s: Abstand
- T1: erster Schwellenwert
- T2: zweiter Schwellenwert
- U: Übertragungscharakteristik
- V: Verfahren
- V1: erster Verfahrensschritt
- V2: zweiter Verfahrensschritt
- V3: dritter Verfahrensschritt
- V4: vierter Verfahrensschritt
- V5: fünfter Verfahrensschritt
- V6: sechster Verfahrensschritt
- V7: siebter Verfahrensschritt
- V8: achter Verfahrensschritt
- V9: neunter Verfahrensschritt
- V10: zehnter Verfahrensschritt
- Z: Zustandscharakteristik
- Z1: erste Zustandscharakteristik
- Z2: zweite Zustandscharakteristik

## Patentansprüche

1. Verfahren zur Ermittlung eines Zustands eines Lichtwellenleiters (23) eines elektrochirurgischen Instruments (12), das wenigstens eine Elektrode (13) aufweist, wobei das elektrochirurgische Instrument (12) zur Versorgung der wenigstens einen Elektrode (14) mit elektrischer Energie mit einer Energiequelle (19) eines Versorgungsgeräts (11) elektrisch verbindbar ist und der Lichtwellenleiter (23) mit einer Auswerteeinheit (27) optisch verbindbar ist, umfassend:
(a) Bereitstellen eines elektrochirurgischen Instruments (12) im Ausgangszustand und Verbinden des elektrochirurgischen Instruments (12) mit der Energiequelle (19) und der Auswerteeinheit (27),
(b) erstes Ermitteln einer Übertragungscharakteristik (U) als Referenzcharakteristik (R) durch die Schritte:
S1 Emittieren von Emissionslicht (Le) einer Lichtquelle (19) der Auswerteeinheit (27) und zumindest teilweises Einkoppeln des Emissionslichts (Le) in ein proximales Ende (24) des Lichtwellenleiters (23),
S2 Empfangen von Empfangslicht (Lr) in der Auswerteeinheit (27), das gebildet wird durch Übertragen des Emissionslichts (Le) über das proximalen Ende (24) zum distalen Ende (25) des Lichtwellenleiters (23), zumindest teilweises Reflektieren am distalen Ende (24) des Lichtwellenleiters (23) und Übertragen vom distalen Ende (25) über das proximale Ende (24) des Lichtwellenleiters (23) zur daran gekoppelten Auswerteeinheit (27),
S3 Ermitteln der Übertragungscharakteristik (U) basierend auf dem Emissionslicht (Le) und dem Empfangslicht (Lr),
(c) Aktivieren des elektrochirurgischen Instruments (12), wobei die wenigstens eine Elektrode (13) mit elektrischer Energie der Energiequelle (19) versorgt wird,
(d) Deaktivieren des elektrochirurgischen Instruments (12) durch Abschalten der Versorgung der wenigstens eine Elektrode (13) mit elektrischer Energie,
(e) erneutes Ermitteln einer Übertragungscharakteristik (U) als Zustandscharakteristik (Z) gemäß den Schritten S1 bis S3,
(f) Vergleichen der Referenzcharakteristik (R) mit der Zustandscharakteristik (Z) zur Ermittlung des aktuellen Zustands des Lichtwellenleiters (23).

2. Verfahren nach Anspruch 1, wobei nach jeder Aktivierung und Deaktivierung des elektrochirurgischen Instruments (12) eine Zustandscharakteristik (Z) und der aktuelle Zustand des Lichtwellenleiters (23) ermittelt wird.

3. Verfahren nach Anspruch 1 oder 2, wobei Licht, das durch Funken- oder Lichtbogenbildung an der wenigstens einen Elektrode (13) entsteht, zumindest teilweise am distalen Ende (25) des Lichtwellenleiters (23) empfangen und an die Auswerteeinheit (27) übertragen wird.

4. Verfahren nach einem der vorhergehenden Ansprüche, wobei die Lichtquelle (34) nur dann zum Emittieren von Emissionslicht (Le) aktiviert wird, wenn keine elektrische Energie zur wenigstens einen Elektrode (13) übertragen wird.

5. Verfahren nach einem der vorhergehenden Ansprüche, wobei das Emissionslicht (Le) der Lichtquelle (34) Lichtwellenlängen (λ) im Bereich kleiner als 380 nm aufweist.

6. Verfahren nach einem der vorhergehenden Ansprüche, wobei das Emissionslicht (Le) der Lichtquelle (34) mehrere Maxima (M1, M2) bei unterschiedlichen Lichtwellenlängen (λ) aufweist.

7. Verfahren nach einem der vorhergehenden Ansprüche, wobei die Auswerteeinheit (27) außerdem eine Lichtanalyseeinheit (28) aufweist, wobei die Lichtquelle (34) an einen ersten Anschluss (26a) einer Kopplungseinheit (26), die Lichtanalyseeinheit (28) an einen zweiten Anschluss (26b) der Kopplungseinheit (26) und der Lichtwellenleiter (23) an einen dritten Anschluss (26c) der Kopplungseinheit (26) angeschlossen sind.

8. Verfahren nach Anspruch 7, wobei der erste Anschluss (26a) und der zweite Anschluss (26b) der Kopplungseinheit (26) optisch voneinander getrennt und separat optisch mit dem dritten Anschluss (26c) der Kopplungseinheit (26) verbunden sind.

9. Verfahren nach Anspruch 8, wobei durch Einkoppeln von Emissionslicht (Le) am ersten Anschluss (26a) der Kopplungseinheit (26) durch den Empfang von Empfangslicht (Lr) in der Auswerteeinheit (27) festgestellt wird, dass der Lichtwellenleiter (23) des elektrochirurgisches Instrument (12) an den dritten Anschluss (26c) der Kopplungseinheit (26) angeschlossen ist.

10. System (10), das insbesondere zur Durchführung des Verfahrens nach einem der vorhergehenden Ansprüche eingerichtet ist, aufweisend eine Energiequelle (19) eines Versorgungsgeräts (11), eine Auswerteeinheit (27) und ein elektrochirurgisches Instrument (12),
wobei die Auswerteeinheit (27) eine Lichtquelle (34) und eine Lichtanalyseeinheit (28) aufweist,
wobei das elektrochirurgische Instrument (12) wenigstens eine Elektrode (13) aufweist, die mit der Energiequelle (19) elektrisch verbunden ist,
wobei das elektrochirurgische Instrument (12) einen Lichtwellenleiter (23) aufweist, der mittels einer Kopplungseinheit (26) optisch mit der Auswerteeinheit (27) gekoppelt ist, wobei die Lichtquelle (34) an einen ersten Anschluss (26a) der Kopplungseinheit (26), die Lichtanalyseeinheit (28) an einen zweiten Anschluss (26b) der Kopplungseinheit (26) und der Lichtwellenleiter (23) an einen dritten Anschluss (26c) der Kopplungseinheit (26) angeschlossen sind, und wobei der erste Anschluss (26a) und der zweite Anschluss (26b) der Kopplungseinheit (26) optisch voneinander getrennt und separat optisch mit dem dritten Anschluss (26c) der Kopplungseinheit (26) verbunden sind.

11. System nach Anspruch 10, wobei die Kopplungseinheit (26) ein Faserbündel (35) mit wenigstens einer ersten optischen Faser (36) und wenigstens einer zweiten optischen Faser (37) aufweist, wobei die wenigstens eine erste optische Faser (36) den ersten Anschluss (26a) der Kopplungseinheit (26) optisch mit dem dritten Anschluss (26c) der Kopplungseinheit (26) verbindet und die wenigstens eine zweite optische Faser (37) den zweiten Anschluss (26b) der Kopplungseinheit (26) optisch mit dem dritten Anschluss (26c) der Kopplungseinheit (26) verbindet.

12. System nach Anspruch 11, wobei die wenigstens eine erste optische Faser (36) und die wenigstens eine zweite optische Faser (37) eine kleinere Kernquerschnittsfläche aufweisen als der Lichtwellenleiter (23).

13. System nach Anspruch 11 oder 12, wobei eine Kernquerschnittsfläche der wenigstens einen zweiten optischen Faser (37) maximal 25% einer Kernquerschnittsfläche des Lichtwellenleiters (23).

14. System nach einem der Ansprüche 11 bis 13, wobei eine Kernquerschnittsfläche der wenigstens einen ersten optischen Faser (26) kleiner ist als eine Kernquerschnittsfläche der wenigstens einen zweiten optischen Faser (37).

15. System nach einem der Ansprüche 10 bis 14, außerdem aufweisend eine Anordnung zur Modenmischung (38) zwischen der Auswerteeinheit (27) und er Kopplungseinheit (26) .
